# FASCICULE DE BREVET EUROPEEN

(11) **EP 3 081 186 B1**
(45) Date de publication et mention de la délivrance du brevet: **23.05.2018**
(21) Numéro de dépôt: 15163782.4
(22) Date de dépôt: 16.04.2015
(51) Int. Cl.: A61C 3/02, B23C 5/10

(54) **INSTRUMENT DE COUPE, NOTAMMENT DENTAIRE**
SCHNEIDEWERKZEUG, INSBESONDERE ZAHNÄRZTLICHES SCHNEIDWERKZEUG
CUTTING INSTRUMENT, IN PARTICULAR A DENTAL CUTTING INSTRUMENT

(43) Date de publication de la demande: 19.10.2016
(73) Titulaire: DENTSPLY SIRONA Inc., York, PA 17401 (US)
(72) Inventeur: Delval, Alain, 1348 Le Brassus (CH)
(74) Mandataire: Micheli & Cie SA

(56) Documents cités:
- US-A- 5 882 198
- US-A1- 2011 195 377

## Description

La présente invention se rapporte au domaine des instruments de coupe et notamment des instruments destinés au domaine médical comme les instruments dentaires ou chirurgicaux. Elle vise plus particulièrement un instrument de coupe rotatif tel qu'une fraise.

Une fraise peut être par exemple utilisée dans le domaine médical pour la chirurgie osseuse ou dans le domaine dentaire pour l'implantologie ou lors de traitements endodontiques pour l'alésage de canaux radiculaires, le façonnage de moignons, la préparation et le taillage de couronnes, la préparation de cavités, l'enlèvement de la dentine, de l'email ou de matériaux de restauration de la dent comme des amalgames, des composites, des céramiques ou des métaux.

Dans le domaine dentaire et en chirurgie osseuse, il est connu d'utiliser des fraises dont au moins la partie active de coupe est en carbure métallique et notamment en carbure de tungstène. Ce matériau est notamment utilisé pour ses performances de coupe et pour sa durabilité. En effet, contrairement à l'acier au carbone, le carbure ne se corrode pas et est assez dur pour être utilisé à grande vitesse même pour couper l'os. Parmi les instruments de coupe dont la partie active est en carbure, on trouve des instruments dont le manche est en acier inoxydable ou des instruments dont l'ensemble formé de la partie active et du manche est venu d'une seule pièce en carbure.

Le but de la présente invention est de réaliser un instrument de coupe notamment un instrument de coupe à usage dentaire ou chirurgical de type fraise dont la partie active est en carbure, en particulier en carbure de tungstène, et dont les performances de coupe sont accrues.

La présente invention a pour objet un instrument de coupe de type rotatif, notamment de type fraise, comprenant une partie active de coupe et un manche, ladite partie active étant en carbure métallique, caractérisé par le fait que le manche est en alliage de nickel-titane; et par le fait que l'instrument comprend en outre une partie intermédiaire en acier inoxydable placée entre la partie active et le manche, ladite partie intermédiaire étant brasée ou soudée à ladite partie active et audit manche.

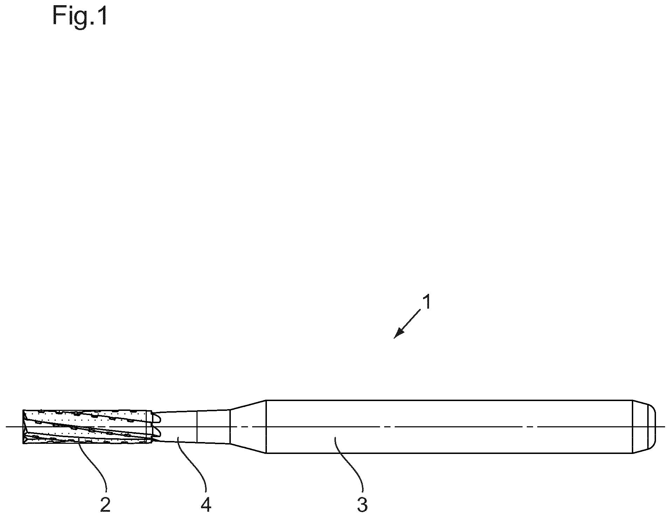

La figure 1 illustre un mode d'exécution de l'invention ayant la forme d'une fraise à usage dentaire.

Selon un mode d'exécution privilégié de l'invention, l'instrument de coupe est un instrument dentaire et notamment une fraise 1 utilisée en implantologie ou lors de traitements endodontiques pour l'alésage de canaux radiculaires, le façonnage de moignons, la préparation et le taillage de couronnes, la préparation de cavités, l'enlèvement de la dentine, de l'email ou de matériaux de restauration de la dent comme des amalgames, des composites, des céramiques ou des métaux. En variante, l'instrument de coupe selon l'invention peut également être un instrument chirurgical destiné par exemple à la chirurgie osseuse.

La fraise 1 comprend une partie active 2 et un manche 3 destiné à être inséré dans un moteur, dans une pièce à main ou un contre-angle pour son entraînement en rotation. La forme de la partie active 2, ses arêtes de coupe, sa longueur, sa géométrie etc. ne seront pas décrites plus en détail ici.

Selon l'invention, la partie active 2 est réalisée en carbure métallique et de préférence en carbure de tungstène (WC) tandis que le manche 3 est réalisé en un alliage de nickel-titane (NiTi). Les alliages de nickel-titane sont très utilisés dans le domaine médical et dentaire, notamment pour leur flexibilité. La composition de l'alliage formant le manche 3 ne sera donc pas décrite plus en détail ici.

Selon l'invention, une partie intermédiaire 4 est intercalée entre la partie active 2 et le manche 3 de l'instrument de coupe 1. Cette partie intermédiaire 4 est réalisée en acier inoxydable et est reliée métallurgiquement de façon appropriée au manche 3 en nickel-titane et à la partie active 2 en carbure. De préférence, la partie intermédiaire 4 est brasée à la partie active 2 en carbure tandis qu'elle est soudée au manche 3 en nickel-titane. De manière générale, la partie intermédiaire 4, la partie active 2 et le manche 3 sont reliés métallurgiquement entre eux par tout moyen approprié connu de l'homme du métier.

La partie intermédiaire 4 a pour but de permettre la liaison entre la partie active 2 en carbure de tungstène et le manche 3 en nickel-titane. En effet, il est très difficile de former une liaison métallurgique directement entre le carbure de tungstène et le nickel-titane.

De préférence, la partie intermédiaire 4 en acier inoxydable présente une longueur ou épaisseur comprise entre 0.05 et 50 mm, plus favorablement de 1,5 mm. L'homme du métier est à même de déterminer la longueur/épaisseur appropriée de la partie intermédiaire 4 permettant une liaison efficace entre la partie active 2 en carbure et le manche 3 en nickel-titane.

L'instrument selon la présente invention présente des performances de coupe accrues, notamment par rapport aux instruments dont le manche est en acier ou en carbure : en effet, le manche 3 en nickel-titane est plus flexible qu'un manche en acier ou en carbure. Cette flexibilité permet notamment de réaliser des instruments de coupe destinés à la chirurgie dont le manche est suffisamment souple pour travailler dans un canon coudé. De plus, la flexibilité du manche engendre des vibrations le long de l'instrument lors de son utilisation (mise en rotation), lesdites vibrations augmentant les performances de coupe de l'instrument.

On réalise ainsi un instrument de coupe, destiné notamment à un usage dans le domaine dentaire ou chirurgical, qui est performant et qui combine les avantages d'une partie active en carbure et d'un manche en nickel-titane.

## Revendications

1. Instrument de coupe (1) de type rotatif, notamment de type fraise, comprenant une partie active (2) de coupe et un manche (3), ladite partie active (2) étant en carbure métallique, **caractérisé par le fait que** le manche (3) est en alliage de nickel-titane ; et **par le fait que** l'instrument comprend en outre une partie intermédiaire (4) en acier inoxydable placée entre la partie active (2) et le manche (3), ladite partie intermédiaire (4) étant brasée ou soudée à ladite partie active (2) et audit manche (3).

2. Instrument selon la revendication 1 **caractérisé par le fait que** la partie active (2) est en carbure de tungstène.

3. Instrument selon la revendication précédente, **caractérisé par le fait que** la partie intermédiaire (4) est soudée au manche (3) et brasée à la partie active (2).

4. Instrument selon l'une des revendications précédentes, **caractérisé par le fait que** la partie intermédiaire (4) a une longueur comprise entre 0.05 et 50 mm.

5. Instrument selon la revendication précédente, **caractérisé par le fait que** la partie intermédiaire (4) a une longueur de 1.5mm.

## Patentansprüche

1. Schneidwerkzeug (1) vom drehbaren Typ, insbesondere vom Typ Fräser, das einen aktiven Schneidteil (2) und einen Griff (3) umfasst, wobei der aktive Teil (2) aus Metallcarbid besteht, **dadurch gekennzeichnet, dass** der Griff (3) aus Nickel-Titan-Legierung besteht; und dadurch, dass das Werkzeug ferner einen Zwischenteil (4) aus Edelstahl umfasst, das zwischen dem aktiven Teil (2) und dem Griff (3) platziert ist, wobei der Zwischenteil (4) an den aktiven Teil (2) und an den Griff (3) gelötet oder geschweißt ist.

2. Werkzeug nach Anspruch 1, **dadurch gekennzeichnet, dass** der aktive Teil (2) aus Wolframcarbid besteht.

3. Werkzeug nach dem vorhergehenden Anspruch, **dadurch gekennzeichnet, dass** der Zwischenteil (4) an den Griff (3) geschweißt und an den aktiven Teil (2) gelötet ist.

4. Werkzeug nach einem der vorhergehenden Ansprüche, **dadurch gekennzeichnet, dass** der Zwischenteil (4) eine Länge aufweist, die zwischen 0,05 und 50 mm enthalten ist.

5. Werkzeug nach dem vorhergehenden Anspruch, **dadurch gekennzeichnet, dass** der Zwischenteil (4) eine Länge von 1,5 mm aufweist.

## Claims

1. Rotary cutting instrument (1), in particular of the bur type, comprising an active cutting part (2) and a handle (3), said active part (2) being made of a metal carbide, **characterised in that** the handle (3) is made of a nickel-titanium alloy; and **in that** the instrument further comprises an intermediate part (4) made of stainless steel placed between the active part (2) and the handle (3), said intermediate part being brazed or welded to said active part (2) and to said handle (3).

2. Instrument as claimed in claim 1, **characterised in that** the active part (2) is made of tungsten carbide.

3. Instrument as claimed in the preceding claim, **characterised in that** the intermediate part (4) is welded to the handle (3) and brazed to the active part (2).

4. Instrument as claimed in any one of the preceding claims, **characterised in that** the intermediate part (4) has a length between 0.05 and 50 mm.

5. Instrument as claimed in the preceding claim, **characterised in that** the intermediate part (4) has a length of 1.5 mm.
